Europäisches Patentamt

European Patent Office

Office européen des brevets

(19)

(11) Publication number: **0 404 590**
**A2**

# EUROPEAN PATENT APPLICATION

(12)

(21) Application number: **90306850.0**

(22) Date of filing: **22.06.90**

(51) Int. Cl.⁵: **G01N 33/96, G01N 33/84**

---

(30) Priority: **22.06.89 DE 3920364**

(43) Date of publication of application:
**27.12.90 Bulletin 90/52**

(84) Designated Contracting States:
**BE CH DE ES FR GB IT LI NL SE**

(71) Applicant: **TECHNICON GMBH**
**Im Rosengarten 11**
**D-6368 Bad Vilbel 1(DE)**

(72) Inventor: **Begemann, Klaus**
**Heinrich-Steih-Strasse 8**
**D-6367 Karben - Rendel(DE)**

(74) Representative: **Wain, Christopher Paul et al**
**A.A. THORNTON & CO. Northumberland**
**House 303-306 High Holborn**
**London WC1V 7LE(GB)**

---

(54) Method of and reagent for the prevention of turbidity.

(57) Turbidity caused by protein precipitation in biological fluids such as serum, plasma or other fluids containing proteins in solution, which fluids are to be subjected for example to colorimetric or other analysis, can be prevented or reduced by adding to the sample urea or a urea derivative in addition to a surface-active agent.

EP 0 404 590 A2

EP 0 404 590 A2

# METHOD OF AND REAGENT FOR THE PREVENTION OF TURBIDITY

This invention relates to a method of, and to a reagent for, reducing or preventing turbidity in liquid samples of serum, plasma and other fluids containing proteins in solution in clinical chemistry, particularly in samples with extreme protein concentrations and protein compositions.

Clinical chemistry often involves working with serum and/or plasma samples, and measuring them by photometry. As is known, photometric measurements are adversely affected by sample turbidity, and turbidity can be caused by precipitation of proteins in the samples. Most of the proteins are ampholytes. Insofar as they are soluble, they form colloidal solutions from which the proteins can be precipitated by neutral salts. The precipitated colloid can be re-dissolved. The solubility of the proteins depends both on the concentration of hydrogen ions and on the salt content of the solution; it is lowest at the isoelectric point. When the pH values are low, as required for some photometric determinations to achieve a reliably measurable coloration, the proteins are often denatured irreversibly. The resulting colloids cause different extinction variations of the measuring wavelength, depending on concentration and type of the denatured proteins, and these variations give rise to incorrect measurements.

Turbidity due to protein precipitation does regularly occur in connection with certain rare diseases, such as plasmacytose, and in plasma samples. The proteins concerned are called paraproteins.

One way of avoiding such interferences is to precipitate and separate the proteins from the samples prior to performing the photometric measurement [Anal. Biochem. 40, 450 (1971)]. This procedure is, however, complicated and unsuited for automated operation. Another approach has been to add surface-active agents to the samples, without separation of the proteins [DE 29 43 423; Clin. Chem. 30, 975 (1984)]. However, this technique is not successful with most plasma samples nor with samples having extreme protein concentrations and protein compositions.

The problem of turbidity can be severe even in systems of very high sample volume where the turbidity is weak per se. It may still be a significant proportion of the total extinction compared to the photometric reaction and thereby make the whole measurement incorrect. Some analysis systems which are available on the market work with fixed ratios of sample volume to reagent volume and these are therefore susceptible to turbidity interferences. Moreover, there are some analysis systems which are more sensitive than others to turbidity because of the particular optical path in the photometer.

Further, the problem of turbidity has been shown to occur frequently in a recently introduced analysis system based on the principle of capsule chemistry (Capsule Chemistry Technology for High Speed Clinical Chemistry Analyses - firm leaflet of Technicon Instruments Corporation, Tarrytown, N.Y., 1985). This instrument is marketed by Technicon as the Technicon CHEM 1 System. In this method, sample and reagent are encapsulated by a film of an inert liquid and transported in this form in a conduit to incubation, mixing, reaction and measuring stations. The system is particularly sensitive to turbidities, possibly because of the optical path of the rays and the very thin layer of the cylindric cuvettes of the photometer (thesis submitted for a diploma by M. Schmidt, Fachhochschule Frankfurt a.M., 1988 "Modellrechnungen an Zylinderkuvetten"). The problem of turbidity interference occurred especially in analyses with low color extinction, e.g. in the colorimetric iron determination. It is known in the colorimetric determination of iron to use urea, or a derivative thereof such as guanidine, to separate the iron from the serum transferrin. Other analysis machines such as the Technicon RA-XT and RA-100 can also be sensitive to sample turbidity.

We have now devised a way of reducing or preventing turbidity in protein-containing samples such as serum, plasma or other fluids. The invention is particularly, but not exclusively, useful in analyses, especially in continuous flow analyses [S. Amer. J. Clin. Path, 28, 311 to 322 (1957)] including capsule chemistry, and where high protein concentrations and extreme compositions exist in the sample, without the need to remove the protein from the sample prior to the analysis.

In our investigations, we have found that, very surprisingly, if a known protein precipitant such as trichloroacetic acid, perchloric acid or uranyl acetate, is added to a serum or plasma sample containing urea (or a urea derivative) and a surface active agent, no proteins are precipitated. Thus, the presence of urea (or a urea derivative) in a mixture of the serum or plasma with a surfactant, has the highly unexpected and advantageous effect of preventing the formation of any significant turbidity.

According to the present invention, there is provided a method of reducing or substantially preventing turbidity in serum, plasma or other fluids containing proteins in solution, wherein a surface-active agent is added to the fluid, characterised in that there is also added urea or a urea derivative.

The invention also includes a reagent mixture for addition to a protein-containing fluid to prevent or reduce turbidity, which reagent is characterised in that it comprises a surface-active agent and urea or a urea derivative, optionally with one or more other additives, the reagent optionally being an aqueous

2

solution.

The preferred surface-active agents are those whose use is known in biochemical analyses, such as for example ethoxylates of 4-(1,1,3,3-tetramethyl butyl)-phenol (e.g. Triton X-100 [R]) or benzyltrimethyl ammonium hydroxide (e.g. Triton B[R]). It is also possible to use other surface-active agents such as p-tert. octyl phenoxy polyethoxy ethanol, polyoxyethylene ether of lauryl alcohol, polyethylene sorbitan monolaurate, polyoxyethylene-14-laurate or polyethylene sorbitan monopalmitate, for example.

The preferred derivatives of urea are thio-urea and guanidine (imino urea) and its acid addition salts. Another urea derivative which can be used is methyl urea.

The combination of urea (or a derivative), and a surface-active agent can be added to the serum or plasma sample to be analyzed in the form of a premixed reagent mixture which advantageously may also contain other auxiliary agents and/or reagents required for the particular analysis. For instance, for iron determination, the reagent mixture could further contain a reducing agent, such as hydroxylamine, ascorbic acid or thioglycolic acid, a complexing agent for copper, such as thiosemicarbazide or 2,9-dimethyl-1,10-phenanthroline (neocuproine) and, if necessary, a buffer such as sodium acetate. For the determination of phosphate and chloride, an acid can be included in the mixture of urea or urea derivative and surface active agent.

The preferred amount of urea or urea derivative is from 1.0 to 5.0 mol/l final concentration. The preferred amount of surface active agent such as those marketed under the trademark Triton X-100 is from 10 to 50 g/l final concentration. The volume ratio of sample to reagent of the invention is preferably from 1:10 to 1:20, most preferably about 1:14.

The reagents of the present invention are especially useful in photometric analyses. In such analyses using a reagent of the invention, the colorimetric determination can be effected (for example in an automated analyzer) by determining the blank in a known manner with a mixture of one part of the sample and of the reagent of the invention (blank reagent), and by mixing the other part of the sample with a color-generating reagent dissolved in the blank reagent. The absorption and extinction of the blank are subtracted from the color-generating mixture in the usual way. This procedure can be followed, for example, with an iron determination using a triacine derivative, such as "Ferene S" or "Ferrozin", and with a chloride determination, using an iron(III) nitrate. In other cases, for example colorimetric phosphate or albumin determination, the color reagent can be added to the sample initially, in admixture with the surface-active agent and the urea or urea derivative, and also with any auxiliary and/or other reagents.

The invention is especially useful in the analysis of liquid samples of serum, plasma or other fluids in various analysis systems including manual as well as discrete analysis systems [Haeckel: Rationalisierung des medizinischen Laboratoriums, pp. 136 to 162 (1979)], continuous-flow analyses and in capsule chemistry analyses.

In order that the invention may be more fully understood, the following Examples are given by way of illustration only.

Example 1:

Determination of the iron concentration in serum samples and heparine plasma samples, i.e. samples wherein the plasma has been stabilised by the addition of heparine to prevent coagulation.

(a) Preparation of the reagents:

Reagent 1 (blank reagent)

108 g imino urea hydrochloride are dissolved in 200 ml of bidistilled water. After the imino urea has dissolved and the solution has reached room temperature, there are added 8 g hydroxylamine hydrochloride, 104 mg neocuproine and 6.25 g Triton X-100[R], which are completely dissolved. Then, the pH is adjusted to 4.5 with 0.1 N caustic soda, and bidistilled water is added to give a total volume of 250 ml.

Reagent 2 (color reagent)

10 mg of Ferene S are dissolved in 10 ml of Reagent 1 (blank reagent).

(b) Analyses

In this Example (as well as in all the other Examples), reagents 1 and 2 were used with the preferred volume ratio of sample to reagent of 1 to 7 each, i.e. 1 volume of the sample was first mixed with 7 volumes of reagent 1 for determining the blank and thereafter additionally mixed with 7 volumes of reagent 2 so that the preferred sample/reagent volume ratio of 1:14 results. No turbidity was detected.

The iron determination was performed in accordance with the invention both manually and by using an automatic analyzing device "TECHNICON CHEM 1". The samples for analysis were plasma with highly pathological protein fractions, and protein concentrations of more than 8.5 g/dl. The results were as follows:

| Sample | CHEM 1[$\mu$g/dl] | Manually [$\mu$g/dl] |
|---|---|---|
| 1 | 134 | 128 |
| 2 | 59 | 61 |
| 3 | 162 | 156 |
| 4 | 76 | 78 |
| 5 | 35 | 39 |
| 6 | 63 | 61 |
| 7 | 66 | 61 |
| 8 | 71 | 73 |
| 9 | 68 | 67 |
| 10 | 39 | 39 |
| 11 | 73 | 61 |
| 12 | 123 | 128 |
| 13 | 95 | 95 |

In the procedure using the CHEM 1 machine, no cuvettes or flow cells are used, but colorimetric and nephelometric effects are measured through the transparent walls of a capillary tube of polytetrafluoroethylene having an internal diameter of e.g. 1.5 mm.

The photometric system comprises a 100 Watt tungsten halogen lamp and 9 monitoring or read-out stations (1 for the blank and 8 for monitoring the development of the final color).

The manual determination was as follows:

400 ul of the plasma sample were mixed with 200 ul of 1.0N hydrochloric acid, and the mixture was allowed to stand for 20 minutes at room temperature. The mixture was then precipitated with 200 ul of trichloroacetic acid (1.2 mol/l). The precipitate was spread by a glass rod and centrifuged for 10 minutes at 3000 revolutions per minute.

Blank, standard and supernatant were pipetted according to the following scheme:

| | Blank | Standard | Sample |
|---|---|---|---|
| Distilled Water | 300 $\mu$l | --- | |
| Aqueous Fe Standard | --- | 300 $\mu$l | |
| Hydrochloric acid 1.4 N | 150 $\mu$l | 150 $\mu$l | |
| Trichloroacetic acid 1.2 mol/l | 150 $\mu$l | 150 $\mu$l | |
| Supernatant | | | 600 $\mu$l |
| Color reagent | 300 $\mu$l | 300 $\mu$l | 300 $\mu$l |
| Color reagent: | | | |
| Ferene S 3 millimol/liter | | | |
| Neocuproine 6 millimol/liter | | | |
| Hydroxylamine 1.2 mol/liter | | | |

After the mixing, extinction of the sample ($E_{sample}$) and extinction of the standard ($E_{standard}$) were measured at 600 nm in a 1 cm cuvette against the blank.

4

$$\text{Iron content (}\mu\text{l/dl) = standard value (}\mu\text{g/dl)} \quad \frac{E_{\text{sample}}}{E_{\text{standard}}}$$

The iron content of plasma samples was also measured in accordance with the invention using the CHEM-1 device as described, and compared with measurements made using a Technicon RA-XT analyzer (not in accordance with the invention). The results were as follows:

|    | CHEM 1[$\mu$g/dl] | RA-XT[$\mu$g/dl] |
|----|-------------------|------------------|
| 1  | 134               | 151              |
| 2  | 59                | 1001 *           |
| 3  | 162               | 1146 *           |
| 4  | 76                | 1054 *           |
| 5  | 35                | 501 *            |
| 6  | 63                | 61               |
| 7  | 71                | 1532 *           |
| 8  | 68                | 1097 *           |
| 9  | 39                | 939 *            |
| 10 | 66                | 1030 *           |

* Turbidity in the measuring cuvette

Using the RA-XT machine, the reagents were:

| Ferene S | 0.93 mmol/l |
|----------|-------------|
| hydroxylamine hydrochloride | 465 mmol/l |
| neocuproine hydrochloride | 4.28 mmol/l |
| buffer | pH 4.55 |
| Measurement preparation: | |
| 25 $\mu$l sample<br>335 $\mu$l reagent | |

A series of serum samples was also analyzed for iron using the method of the invention in a CHEM 1 machine, and using the RA-XT machine (with no urea or urea derivative). The results were as follows:

| | CHEM 1[$\mu$g/dl] | RA-XT[$\mu$g/dl] |
|---|---|---|
| 1 | 126 | 130 |
| 2 | 61 | 71 |
| 3 | 98 | 104 |
| 4 | 53 | 49 |
| 5 | 24 | 1230 * |
| 6 | 63 | 79 |
| 7 | 91 | 730 * |
| 8 | 123 | 136 |
| 9 | 76 | 80 |
| 10 | 96 | 89 |
| 11 | 97 | 85 |
| 12 | 77 | 74 |
| 13 | 137 | 142 |
| 14 | 111 | 1120 * |
| 15 | 43 | 51 |

* turbidity in the measurement cuvette

The reagents were as previously described.

Example 2:

Determination of the phosphate concentrations in serum and heparine plasma samples

Preparation of reagent:

25 g thiourea are dissolved in 200 ml of bidistilled water. After the solution has reached room temperature, 87 mg of ammonium molybdate (color reagent) are dissolved therein and 7 ml concentrated sulfuric acid (98%) are carefully added. Thereafter, 6.5 g of Triton X-100[R] are dissolved therein and bidistilled water is added to give a total volume of 250 ml.

Plasma samples with pathological protein fractions were measured by means of the automatic analyzing device marketed by Technicon Instruments Corporation as the Technicon RA-XT, with sample blank. Saline (0.9%) with wetting agent W was utilized as the sample blank reagent.

The results were as follows:

6

| | CHEM 1[mg/dl] | RA-XT[mg/dl] |
|---|---|---|
| 1 | 3.9 | 4.0 |
| 2 | 2.6 | 2.8 |
| 3 | 4.0 | 4.1 |
| 4 | 3.5 | 3.8 |
| 5 | 3.3 | 3.7 |
| 6 | 3.9 | 4.0 |
| 7 | 4.3 | 5.3 * |
| 8 | 2.6 | 2.3 |
| 9 | 3.9 | 4.0 |
| 10 | 3.1 | 3.3 |

\* turbidity in the measurement cuvette
Reagent used in RA-XT:
ammonium molybdate 0.28 mmol/l
sulfuric acid 130 mmol/l
Measurement preparation:
5 μl sample
325 μl sample

Example 3:

Determination of the albumin concentration in serum and heparine plasma samples

Preparation of the reagent:

75 g of urea are dissolved in 200 ml of bidistilled water. After the solution has reached room temperature, 50 mg of bromocresol green (color reagent) and 6.25 g of Triton X-100 are dissolved successively. The pH is adjusted to 4.2 and bidistilled water is added to give a total volume of 250 ml.

Plasma samples with pathological protein fractions were measured by means of the Technicon automatic analyzing device RA-100.

The results were as follows:

| | CHEM 1[g/dl] | RA-XT[g/dl] |
|---|---|---|
| 1 | 4.3 | 4.5 |
| 2 | 4.8 | 4.7 |
| 3 | 3.7 | 4.1 |
| 4 | 3.1 | 3.2 |
| 5 | 3.8 | 3.7 |
| 6 | 4.3 | 4.1 |
| 7 | 6.3 | 9.6 * |
| 8 | 3.5 | 3.4 |
| 9 | 3.9 | 3.7 |
| 10 | 3.7 | 3.5 |

* Turbidity in the measurement cuvette
Reagent used in RA-100:
bromocresol green 0.30
mmol/l
buffer pH 4.20
Measurement preparation:
2.5 μl sample
375 μl reagent

Example 4:

Determination of the chloride concentration in serum and heparine plasma samples

Preparation of the reagents

Reagent 1 (blank reagent)

108 g of imino urea hydrochloride are dissolved in 200 ml of bidistilled water. After the imino urea has dissolved and the solution has reached room temperature, 5 mg of methane sulfonic acid and 6.25 g of Triton X-100[R] are dissolved therein. After complete dissolution of all the constituents, bidistilled water is added to give a total volume of 250 ml.

Reagent 2 (color reagent)

250 mg iron(III) nitrate nonahydrate are dissolved in 20 ml of Reagent (1) blank reagent.
Plasma samples with pathological protein fractions were measured by means of the Technicon CHEM 1 (capsule chemistry) automatic analyzer, and in a Technicon RA-XT automatic analyzer.
The results were as follows:

|    | CHEM 1[mmol/l] | RA-XT[mmol/l] |
|----|------|------|
| 1  | 101  | 102 |
| 2  | 108  | 107 |
| 3  | 96   | 97  |
| 4  | 99   | 98  |
| 5  | 98   | 99  |
| 6  | 101  | 100 |
| 7  | 106  | xxxxx * |
| 8  | 111  | 109 |
| 9  | 97   | 97  |
| 10 | 95   | 99  |

* Turbidity in the measurement cuvette
Reagent used in RA-XT:
mercury nitrate 0.80 mmol/l
potassium thiocyanate 1.19 mmol/l
iron(III) chloride 35.00 mmol/l
nitric acid 110.00 mmol/l
Measurement preparation: ·
10 µl sample
383 µl reagent

## Claims

1. A method of reducing or substantially preventing turbidity in serum, plasma or other fluids containing proteins in solution, wherein a surface-active agent is added to the fluid, characterized in that there is also added urea or a urea derivative.

2. A method according to claim 1, wherein guanidine or an acid addition salt thereof is used as the urea derivative.

3. A method according to claim 1 or 2, wherein a reagent mixture is added to the sample, which mixture contains the surface-active agent and the urea or urea derivative, and optionally one or more other additives.

4. A method of determining the concentration of a component in serum, plasma or another fluid sample containing protein in solution, by means of manual analysis, discrete analysis, continuous-flow analysis or capsule chemistry analysis, characterised in that the sample is first treated by the method of claim 1,2 or 3 to reduce or prevent turbidity therein.

5. A reagent for use in the method of claim 3, characterised in that it comprises a surface-active agent and urea or a urea derivative, optionally with one or more other additives, the reagent optionally being an aqueous solution.

6. A reagent according to claim 5, for the colorimetric direct determination of iron, which reagent comprises guanidinium chloride as the urea derivative, a reducing agent, a copper masking agent and, optionally, a buffer and/or a triazine derivative.

7. A reagent according to claim 5, for the colorimetric direct determination of phosphate, which reagent contains thiourea as the urea derivative, and sulfuric acid and ammonium molybdate.

8. A reagent according to claim 5, for the colorimetric direct determination of albumin, which reagent contains bromocresol green.

9. A reagent according to claim 5, for the colorimetric direct determination of chloride, which reagent contains guanidinium chloride as the urea derivative, methane sulfonic acid and, optionally, iron(III) nitrate.

10. The use of a reagent according to any of claims 5 to 9 for determining the concentration of a component of serum, plasma or another fluid containing protein in solution, by means of manual analysis, discrete analysis, continuous-flow analysis or capsule chemistry analysis.